# EUROPEAN PATENT SPECIFICATION

(11) **EP 3 922 196 B1**
(45) Date of publication and mention of the grant of the patent: **22.05.2024**
(21) Application number: 21164196.4
(22) Date of filing: 23.03.2021
(51) Int. Cl.: A61B 17/122, A61B 17/128, A61B 17/00

(54) **INTRAOPERATIVE CLIP LOADING DEVICE**
INTRAOPERATIVE CLIP-LADEVORRICHTUNG
DISPOSITIF DE CHARGEMENT DE PINCE INTRAOPÉRATOIRE

(30) Priority: 26.03.2020 US 202063000091 P; 12.01.2021 US 202117146585
(43) Date of publication of application: 15.12.2021
(73) Proprietor: Covidien LP, Mansfield, MA 02048 (US)
(72) Inventor: THOMAS, Justin, New Haven, CT 06512 (US); BARIL, Jacob C., Norwalk, CT 06851 (US); DININO, Matthew A., Newington, CT 06111 (US); PILLETERE, Roy J., Middletown, CT 06457 (US)
(74) Representative: Maschio & Soames IP Ltd

(56) References cited:
- WO-A1-2017/019865
- CN-A- 101 164 502
- US-A1- 2004 097 971
- US-A1- 2014 309 677

## Description

### CROSS-REFERENCE TO RELATED APPLICATION

This application claims the benefit of and priority to U.S. Provisional Patent Application Serial No. 63/000,091, filed March 26, 2020.

### FIELD

This technology is generally related to surgical clip appliers and, more particularly, to a clip loading device for intraoperatively loading ligation clips into a ligation clip applier.

### BACKGROUND

Endoscopic ligation clip appliers are used to apply ligation clips to body vessels during surgical procedures to occlude or partially occlude the body vessels. These clip appliers are inserted through small diameter cannulas or small incisions in a patient's body to access a surgical site within a body cavity. Performing a surgical procedure endoscopically reduces the amount of trauma inflicted on a patient during a surgical procedure to minimize patient discomfort and reduce patient recovery times.

Surgical clip appliers include single-fire clip appliers and multi-fire clip appliers. In single-fire clip appliers, a ligation clip is loaded into the clip applier after each use. Typically, the clip applier is used to withdraw a single clip from a clip package to load the clip into jaws of the clip applier prior to each use of the clip applier. During an endoscopic procedure in which a single-fire clip applier is used, the clip applier is removed from a body cavity after each use to reload a ligation clip into the clip applier. This process is time consuming and increases the possibility of infection, thus increasing trauma to the patient.

Multi-fire clip appliers include a clip cartridge that is coupled to an elongate body of the clip applier that includes a plurality of ligation clips that are sequentially supplied to the jaws of the clip applier to facilitate placement of multiple clips on a body vessel or on body vessels without withdrawing the clip applier from within a body cavity.

These clip appliers are complex especially where articulation of the distal portion of the clip applier is desired.

United States Patent Application US 2004/0097971 A1 discloses a surgical clip applying apparatus comprising an actuator device, a ratchet mechanism, a locking mechanism, and a clip driving device. International Patent Application WO 2017/019865 A1 discloses a surgical clip cartridge including a base portion extending in a longitudinal direction, the base portion having a bottom surface and defining a mounting groove for attaching the base portion onto a surgical instrument shaft.

United States Patent Application US 2014/0309677 A1 discloses a clip holder configured to retain one or more clips or staples.

### SUMMARY

The present invention is defined by the features of the independent claim. Preferred embodiments are given in the dependent claims.

Any methods described herein do not form part of the claimed invention.

Other features of the disclosure will be appreciated from the following description.

### BRIEF DESCRIPTION OF DRAWINGS

Various aspects of the disclosed intraoperative ligation clip loading device are described herein below with reference to the drawings, wherein:
FIG. 1 is a side perspective view of an intraoperative ligation clip loading device according to aspects of the present disclosure;
FIG. 2 is a side perspective view of an exemplary ligation clip of the intraoperative ligation clip loading device shown in FIG. 1;
FIG. 3 is a cross-sectional view of a distal portion an elongate body of the intraoperative ligation clip loading device taken along section line 3-3 of FIG. 1 including a plurality of ligation clips;
FIG. 4 is an enlarged view of the indicated area of detail shown in FIG. 3;
FIG. 5 is a cross-sectional, cutaway view of a central portion of the elongate body of the intraoperative ligation clip loading device shown in FIG. 1 including a plurality of ligation clips;
FIG. 5A is a side perspective view of clip retainers supported within a portion of the elongate body of the intraoperative ligation clip loading device shown in FIG. 1 with the portion of the elongate body shown in phantom;
FIG. 5B is a side perspective view of the clip retainers supported within a portion of the elongate body of the intraoperative ligation clip loading device shown in FIG. 1 with the portion of the elongate body shown in phantom and a clip supported between the clip retainers;
FIG. 6 is a cross-sectional view of the distal portion of the intraoperative ligation clip loading device shown in FIG. 1 as the plurality of ligation clips are advanced within the body of the intraoperative ligation clip loading device;
FIG. 7 is a cross-sectional, cutaway view of the central portion of the body of the intraoperative ligation clip loading device shown in FIG. 1 as the plurality of ligation clips are advanced within the body of the intraoperative ligation clip loading device;
FIG. 8 is a side perspective view of the distal portion of the intraoperative ligation clip loading device shown in FIG. 8A as the ligation clip applier is inserted into the distal portion of the intraoperative ligation clip loading device;
FIG. 8A is a side perspective view of the intraoperative ligation clip loading device shown in FIG. 1 with a ligation clip applier positioned adjacent a distal portion of the loading device;
FIG. 9 is a cross-sectional view taken along section line 9-9 of FIG. 8 illustrating the ligation clip applier positioned within the distal portion of the intraoperative ligation clip loading device;
FIG. 10 is a cross-sectional view taken along section line 10-10 of FIG. 9; and
FIG. 11 is a side perspective view from a distal end of a ligation clip applier supporting a ligation clip removed from the distal portion of the intraoperative ligation clip loading device;
FIG. 12 is a flowchart of the method for using the intraoperative clip loading device shown in FIG. 1;
FIG. 13 is a perspective view of a patient with the intraoperative device shown in FIG.1 and the clip applier shown in FIG. 11 positioned within a patient;
FIG. 14 is a perspective view of a body cavity of the patient shown in FIG. 13 with the intraoperative device shown in FIG. 1 and the clip applier shown in FIG. 11 positioned within the patient as a clip is applied to tissue;
FIG. 15 is a perspective view of the body cavity of the patient shown in FIG. 14 with the intraoperative device shown in FIG. 1 and the clip applier shown in FIG. 11 positioned within the patient as a second clip is loaded into the clip applier; and
FIG. 16 is a perspective view of a body cavity of the patient shown in FIG. 13 with the intraoperative device shown in FIG. 1 and the clip applier shown in FIG. 11 positioned within a patient as the second clip is applied to tissue.

### DETAILED DESCRIPTION

The disclosed intraoperative clip loading device will now be described in detail with reference to the drawings in which like reference numerals designate identical or corresponding elements in each of the several views. However, it is to be understood that aspects of the disclosure included herein are merely exemplary of the disclosure and may be embodied in various forms. Well-known functions or constructions are not described in detail to avoid obscuring the disclosure in unnecessary detail. Therefore, specific structural and functional details disclosed herein are not to be interpreted as limiting, but merely as a basis for the claims and as a representative basis for teaching one skilled in the art to variously employ the disclosure in virtually any appropriately detailed structure. In addition, directional terms such as front, rear, upper, lower, top, bottom, distal, proximal, and similar terms are used to assist in understanding the description and are not intended to limit the disclosure.

In this description, the term "proximal" is used generally to refer to that portion of the device that is closer to a clinician, while the term "distal" is used generally to refer to that portion of the device that is farther from the clinician. In addition, the term "endoscopic" is used generally to refer to endoscopic, laparoscopic, arthroscopic, and/or any other procedure conducted through a small diameter incision or cannula. Further, the term "clinician" is used generally to refer to medical personnel including doctors, nurses, and support personnel.

The disclosed intraoperative clip loading device includes an elongate body that supports a plurality of ligation clips and a clip advancing member for advancing the plurality of ligation clips towards a distal portion of the elongate body. The distal portion of the elongate body defines an opening that is accessible to a ligation clip applier to facilitate loading of the ligation clip applier through the distal portion of the elongate body of the intraoperative clip loading device. The elongate body is dimensioned to be positioned through a standard size port of a cannula to access a surgical site. The loading device can be positioned within a body cavity during an endoscopic surgical procedure to facilitate intraoperative loading of the ligation clip applier *in vivo.*

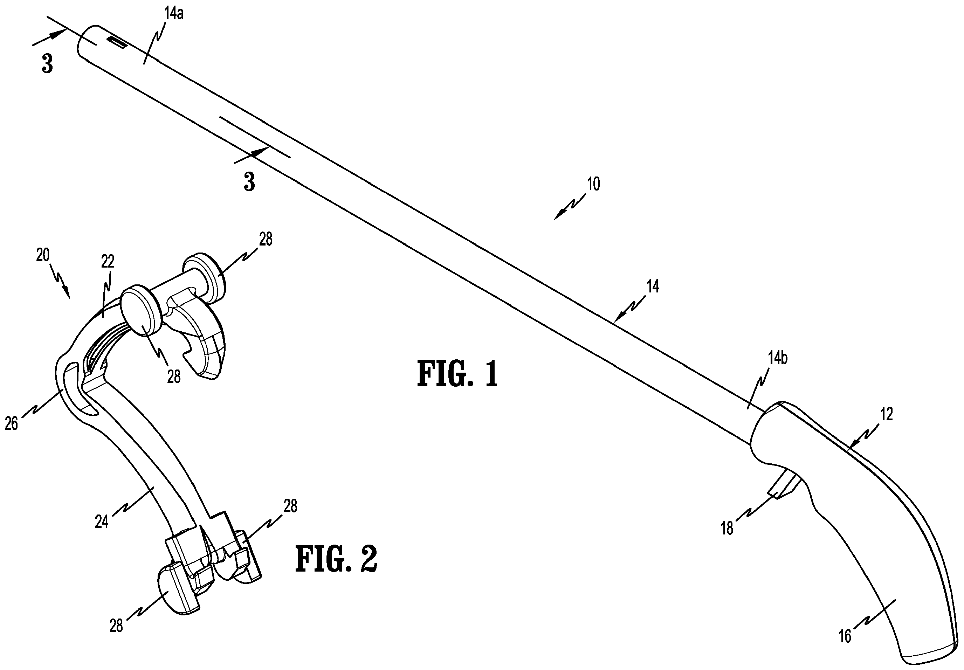

FIG. 1 illustrates exemplary aspects of the disclosed intraoperative clip loading device shown generally as loading device 10. The loading device 10 includes a handle assembly 12 and an elongate body 14. The elongate body 14 includes a distal portion 14a and a proximal portion 14b that is fixedly coupled to the handle 12. The handle assembly 12 includes a handle grip 16 and an actuator 18. Although the handle grip 16 is illustrated as a pistol-type grip, it is envisioned that the handle grip 16 may have a variety of configurations that are ergonomically pleasing to a clinician.

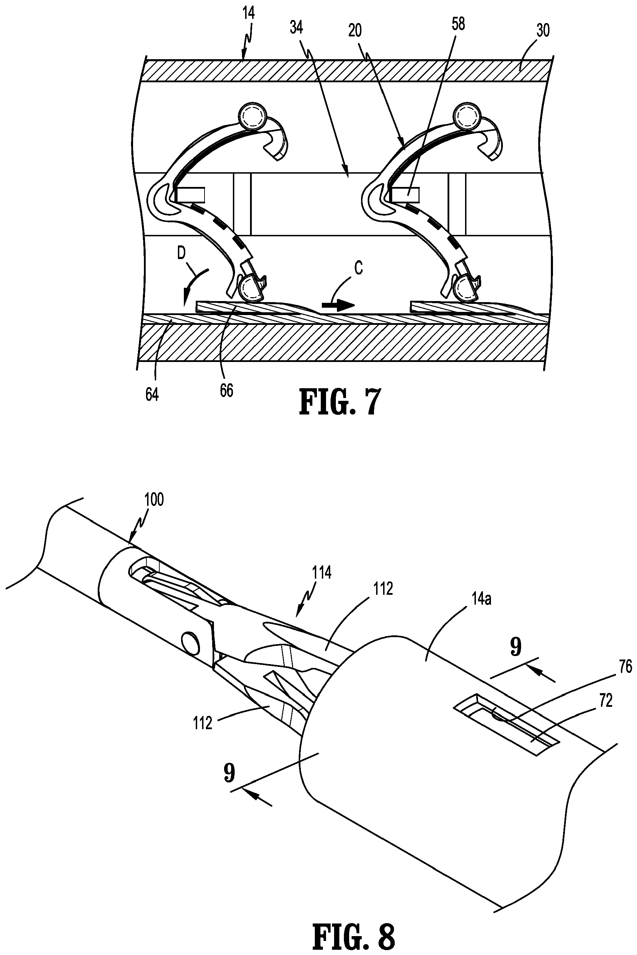

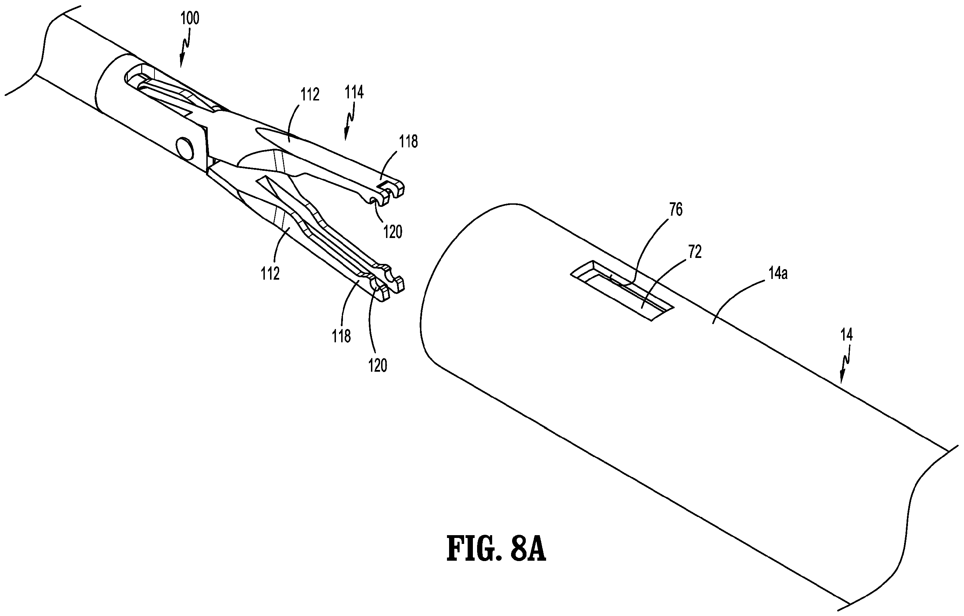

FIG. 2 illustrates a ligation clip 20, a plurality of which is received within the elongate body 14 of the loading device 10. Each of the ligation clips 20 includes a first beam 22, a second beam 24, and a hinge portion 26 that interconnects the first beam 22 to the second beam 24 and allows the ligation clip 20 to pivot between open and clamped positions. Each of the beams includes bosses 28 that are positioned on opposite sides of the respective beams 22 and 24. The bosses 28, as known in the art, provide engagement surfaces that facilitate securement of the ligation clip 20 onto to a ligation clip applier 100 (FIG. 8A).

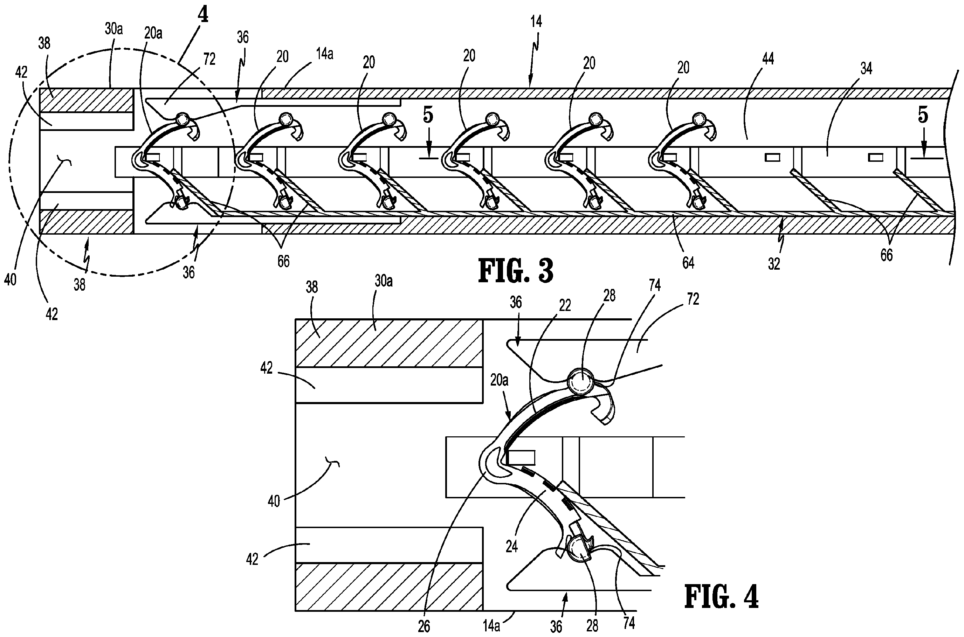

FIGS. 3-5B illustrate the elongate body 14 of the loading device 10 which includes an outer tube 30, a clip advancing member 32, clip retainers 34, clip locking arms 36, and a guide member 38. The outer tube 30 is hollow and houses each of the components of the elongate body 14 listed above as well as a plurality of ligation clips 20. The outer tube 30 includes a distal portion 30a that receives or supports the guide member 38. In aspects of the disclosure, the outer tube 30 and the guide member 38 are integrally formed although it is envisioned that the guide member 38 could be formed separately from the outer tube 30 and secured to the outer tube 30. The guide member 38 defines a through bore 40 and includes diametrically opposed rectangular guide channels 42 that extend from the outwardly from the through bore 40. The guide channels 42 receive jaws 112 (FIG. 8A) of an end effector 114 of a clip applier 100 to guide the jaws 112 into engagement with a distal-most clip 20a of the plurality of clips 20 supported within the outer tube 30 as described in further detail below.

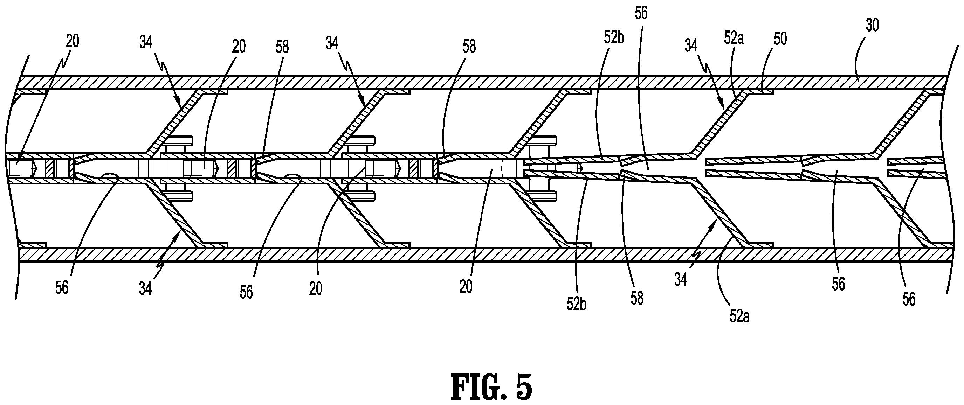

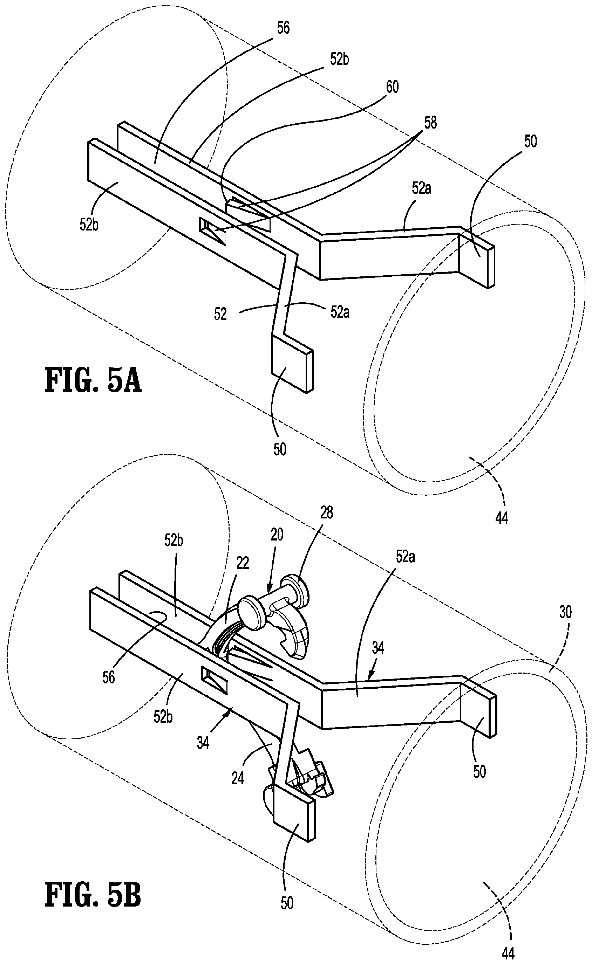

The clip retainers 34, best shown in FIGS. 5-5B, are formed of a resilient material and include a base portion 50 and flexible arms 52. The flexible arms 52 extend from the base portion and include a transverse portion 52a and a longitudinal portion 52b. The base portion 50 of each of the clip retainers 34 is secured to an inner wall 44 of the outer tube 30. Each of the clip retainers 34 is positioned in opposition to another one of the clip retainers 34 to define a clip retention pocket 56 between the two opposed clip retainers 34. The clip retention pockets 56 are defined between respective opposed clip retainers 34 along the outer tube 30 from a proximal end portion of the outer tube 30 to a distal end portion of the outer tube 30. When a ligation clip 20 is received between the longitudinal portions 52b of the two opposed clip retainers 34, the flexible arms 52 of the clip retainers 34 flex outwardly and apply an inwardly directed force onto the hinge portion 26 and beams 22 and 24 of the ligation clip 20 to support the ligation clip 20 within the outer tube 30 of the elongate body 14 of the loading device 10 (FIG. 5B).

Each of the longitudinal portions 52b of the flexible arms 52 of the clip retainers 34 includes a flexible tab 58 that has a proximal end that is secured to the flexible arm 52 in cantilevered fashion and a distal end that defines a distal stop surface 60. When a ligation clip 20 is received between the flexible arms 52, the tabs 58 are deformed inwardly to allow the ligation clip 20 to move distally by the tab 58. When the ligation clip 20 moves past the tab 58, the tab 58 springs outwardly such that the stop surface 60 of the tab 58 is aligned with the hinge portion 26 of a respective one of the ligation clips 20 to obstruct proximal movement of the ligation clip 20 within the outer tube 30.

FIGS. 3 and 4 illustrate the clip advancing member 32 which is movable within the outer tube 30 between retracted and advanced positions and includes an elongate member 64 and a plurality of resilient fingers 66. Each of the plurality of resilient fingers 66 extends from the elongate member 64 in a distal direction at an acute angle into the outer tube 30 and engages the second beam 24 of one of the ligation clips 20. The elongate member 64 has a proximal portion (not shown) that is coupled to the actuator 18 of the handle assembly 12 such that actuation of the actuator 18 moves the clip advancing member 32 between its retracted and advanced positions. When the clip advancing member 32 is moved from its retracted position to its advanced position, each of the ligation clips 20 is advanced within the outer tube 30 from a first retention pocket 56 between two opposed clip retainers 34 to the next distally located retention pocket 56 within the outer tube 30.

The clip locking arms 36 are secured in cantilevered fashion within the distal portion of the outer tube 30 and are formed of a resilient material. Each of the clip locking arms 36 has a proximal portion 70 that is secured to the inner wall 44 of the outer tube 30 and a distal portion 72 that includes a concavity 74. The concavities 74 of the clip locking arms 36 receive the bosses 28 of a respective ligation clip 20 when the ligation clip 20 is delivered to a position between the clip locking arms 36 by the clip advancing member 32. The distal portion 72 of each of the clip locking arms 36 is positioned adjacent to a cutout 76 formed in the outer tube 30 of the elongate body 14. The cutouts 76 accommodate the distal portions of the clip locking arms 36 as a ligation clip 30 is advanced to a position between the clip locking arms 36. More specifically, the cutouts 76 allow the clip locking arms 36 to flex outwardly when a ligation clip 20 is delivered to the clip locking arms 36 by the clip advancing member 32 and when a ligation clip 20 is removed from the elongate body 14 by a clip applier 100 (FIG. 8A) as described in further detail below. The clip locking arms 36 are longitudinally aligned with guide channels 42 formed in the guide member 38 and with the clip retention pockets 56 defined by the clip retainers 34 positioned along the length of the outer tube 30.

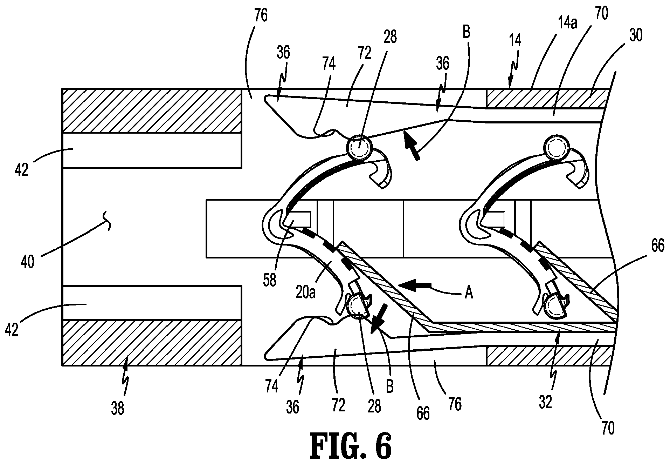

FIG. 6 illustrates the distal portion 14a of the elongate body 14 as the clip advancing member 32 is moved towards its advanced position to advance the plurality of clips 20 within the outer tube 30 of the elongate body 14. When the clip advancing member 32 is moved towards its advanced position in the direction indicated by arrow "A" in FIG. 6, each of the resilient fingers 66 of the clip advancing member 32 engages a respective ligation clip 20 and advances a respective ligation clip 20 from a clip retention pocket 56 to the next distally located clip retention pocket 56 defined between two opposed clip retainers 34. The distal-most clip 20a of the plurality of ligation clips 20 is advanced to a position between the clip locking arms 36. As the distal-most clip 20a is moved between the clip locking arms 36, the bosses 28 on the distal-most clip 20a engage the clip locking arms 36 to deflect the distal portions 72 of the clip locking arms 36 in the direction of arrow "B" into the cutouts 76 formed in the outer tube 30. As the distal-most ligation clip 20a moves to a position in which the bosses 28 of the ligation clip 20a are aligned with the concavities 74 in the clip locking arms 36, the clip locking arms 36 snap inwardly to position the bosses 28 within the concavities 74 to grip the ligation clip 20a between the clip locking arms 36.

As the clip advancing member 32 moves towards it advanced position, and the ligation clips 20 are received in the next distal clip retention pocket 56, the ligation clips 20 pass over one of the flexible tabs 58 of the clip retainers 34. As the ligation clips 20 pass over the flexible tabs 58, the flexible tabs 58 are deformed inwardly until the hinge portion 26 of the ligation clips 20 pass distally over the flexible tabs 58. Once the ligation clips 20 pass over the flexible tabs 58, the flexible tabs 58 return to a positon in which the stop surfaces 60 of the tabs 58 are aligned with the hinge portions 26 of the ligation clips 20 to prevent proximal movement of the ligation clips 20 within the outer tube 30.

FIG. 7 illustrates the clip advancing member 32 as the clip advancing member 32 moves in the direction of arrow "C" from its advanced position back towards its retracted position. As the clip advancing member 32 returns to its retracted position, the resilient fingers 66, which are angled towards the distal end of the outer tube 30, engage the ligation clips 30 positioned proximally of the respective resilient fingers 66. As described above, the ligation clips 20 are prevented from moving proximally within the outer tube 30 by the flexible tabs 58. As such, the resilient fingers 66 of the clip advancing members are deformed inwardly towards the elongate member 64 in the direction indicated by arrows "D" and pass beneath the respective ligation clips 20. In the retracted position of the clip advancing member 32, the resilient fingers 66 return to their undeformed positions shown in FIG. 3 located proximally of the respective ligation clips 20.

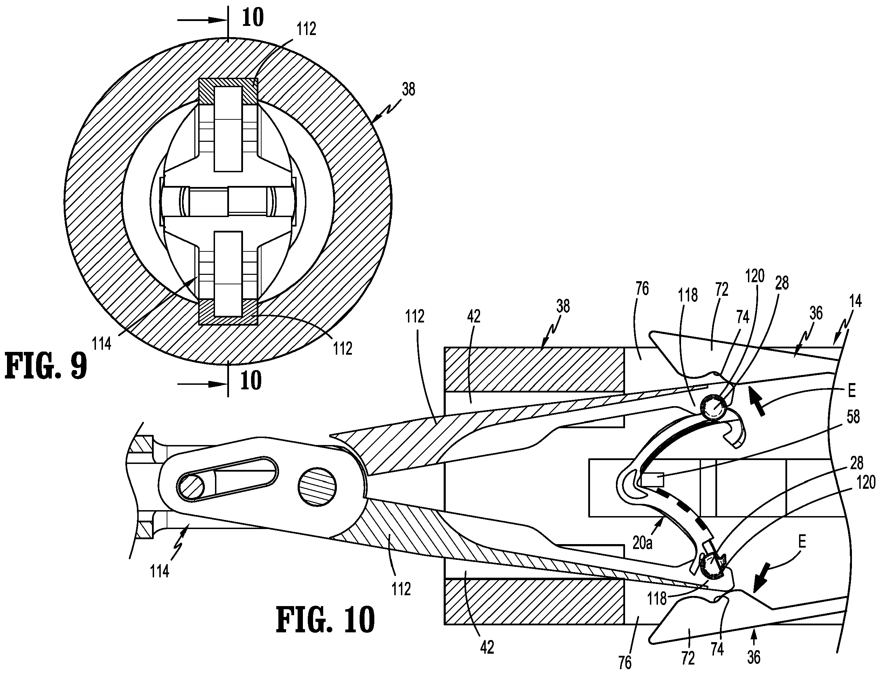

FIGS. 8-11 illustrate the loading device 10 as a ligation clip applier 100 is inserted into the distal portion 14a of the elongate body 14 to remove the distal-most ligation clip 20a (FIG. 10) from the loading device 10. The clip applier 100 includes an end effector 114 that includes jaws 112. The jaws 112 are movable in relation to each other to move a ligation clip 20 from an open position to a clamped position as known in the art. Each of the jaws 112 of the end effector 114 includes a distal portion including a hook portion 118 that defines a recess 120 that receives the bosses 28 of a ligation clip 20 to secure the ligation clip 20 to the clip applier 100.

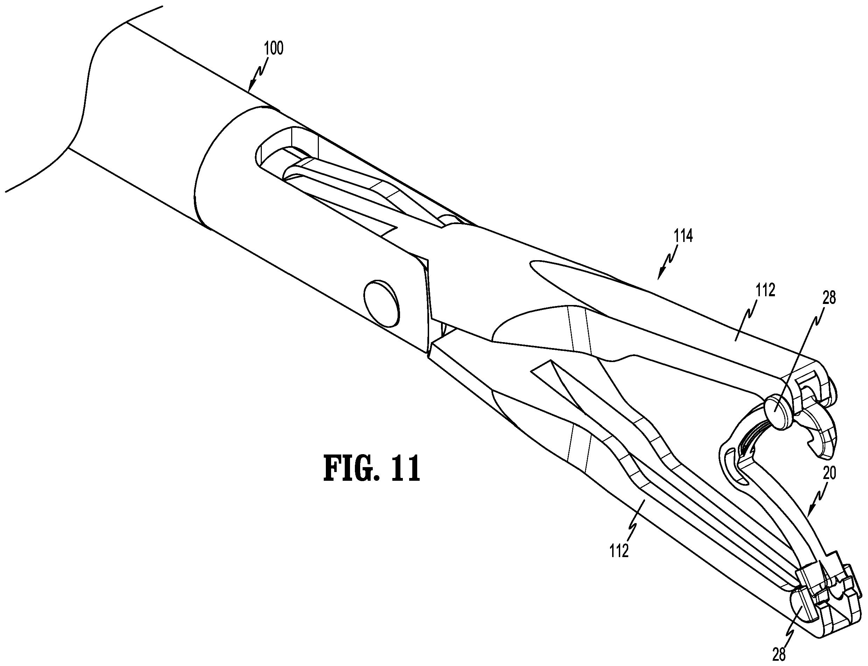

In order to load a ligation clip 20 onto the clip applier 100, the jaws 112 of the clip applier 100 are inserted into the channels 42 of the guide member 38 in the distal portion 14a of the loading device 100. The channels 42 are aligned with the clip retention pockets 56 defined by the clip retainers 34 and with the clip locking arms 36 such that the jaws 112 of the clip applier 100 are aligned with the first and second beams 22 and 24 of the distal-most ligation clip 20a. When the jaws 112 engage the distal portion 72 of the clip locking arms 36, the clip locking arms 36 are deflected outwardly in the direction of arrows "E" in FIG. 10 to release the distal-most ligation clip 20a from the concavities 74 of the clip locking arms 36. As the jaws 112 of the clip applier 100 move further distally into the outer tube 30 of the loading device 10, the jaws 112 engage the beams 22 and 24 of the ligation clip 20a such that the bosses 28 are received in the recesses 120 of the hook portions 118 of the jaws 112. The stop surface 60 of the tab 58 of the clip retainers 34 engage the ligation clip 20a to prevent proximal movement of the ligation clip 20a within the outer tube 30. Once the bosses 28 are received within the recesses 120 of the jaw members 112, the end effector 114 of the clip applier 100 can be removed from the loading device 10 (FIG. 11) with the ligation clip 20 supported on the jaws 112. After the distal-most ligation clip 20a is removed from the elongate body 14 of the loading device 10, the actuator 18 of the handle assembly 12 of the loading device 10 can be actuated to advance the ligation clips 20 within the elongate body 14 to position the distal-most clip 20a in position between the clip locking arms 36 of the loading device 10.

The disclosed loading device 10 can be inserted through a cannula (not shown) to a surgical site within a body cavity to facilitate reloading of a single-use clip applier 100 during a surgical procedure at the surgical site. The elongate body 14 has a length to access the surgical site and to house a plurality of ligation clips 20, e.g., 5 or more ligation clips 20. The loading device 10 obviates any need to remove a single-use clip applier from a body cavity through a cannula to facilitate reloading of the clip applier 100.

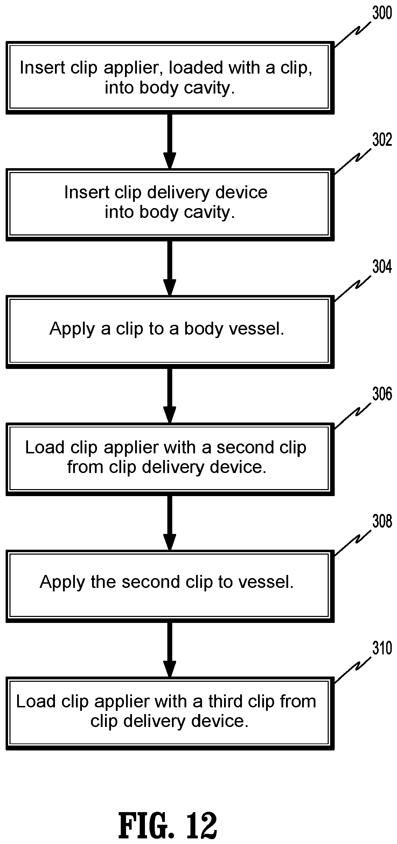

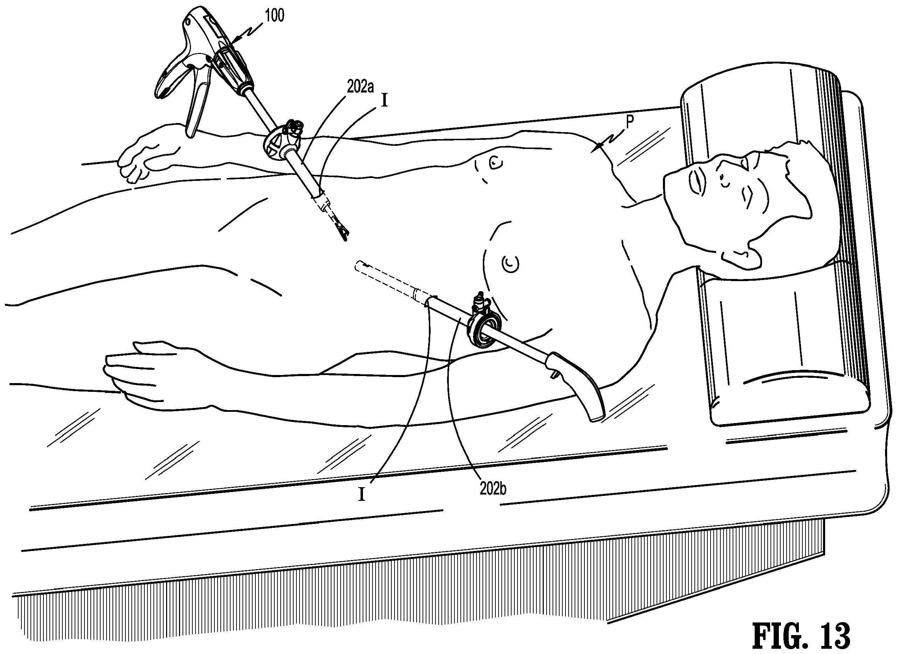

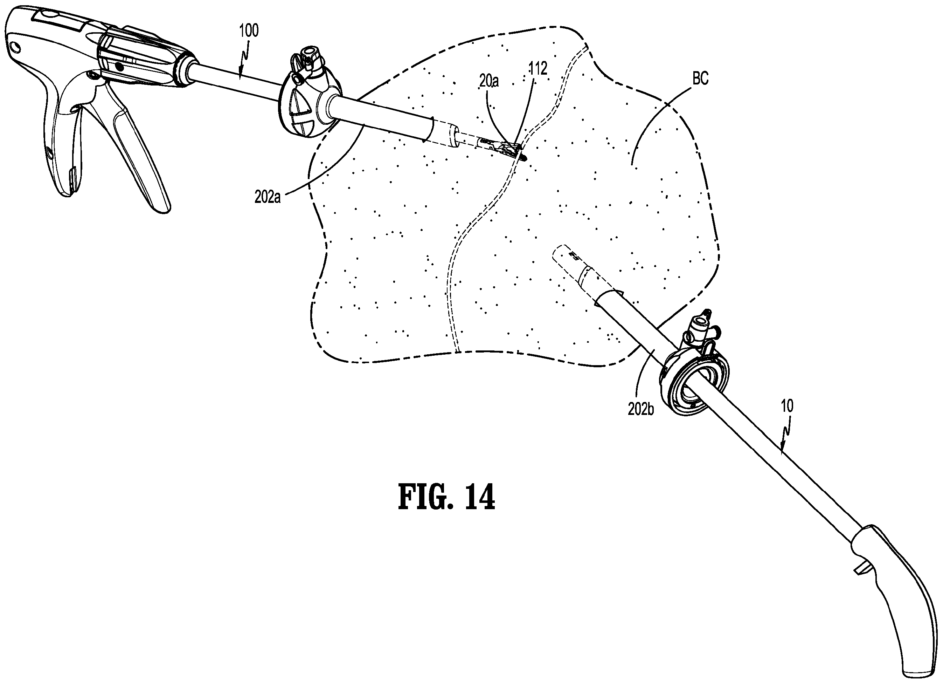

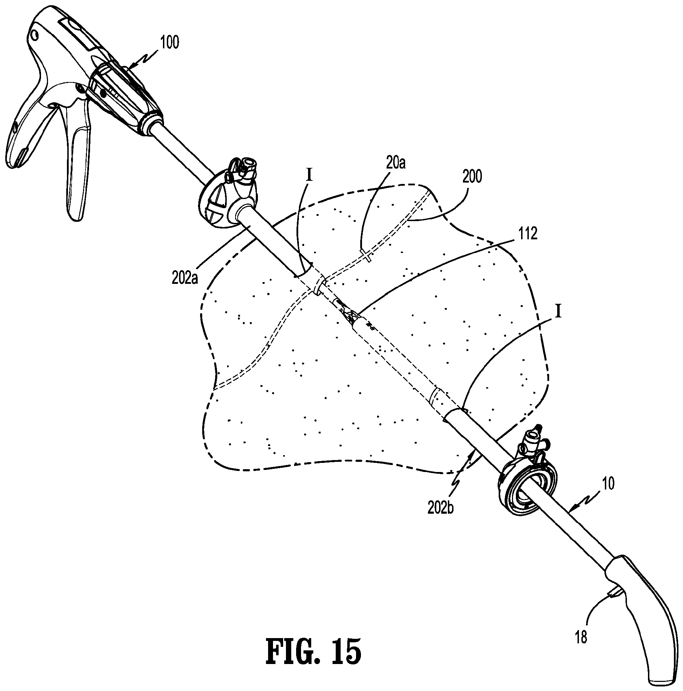

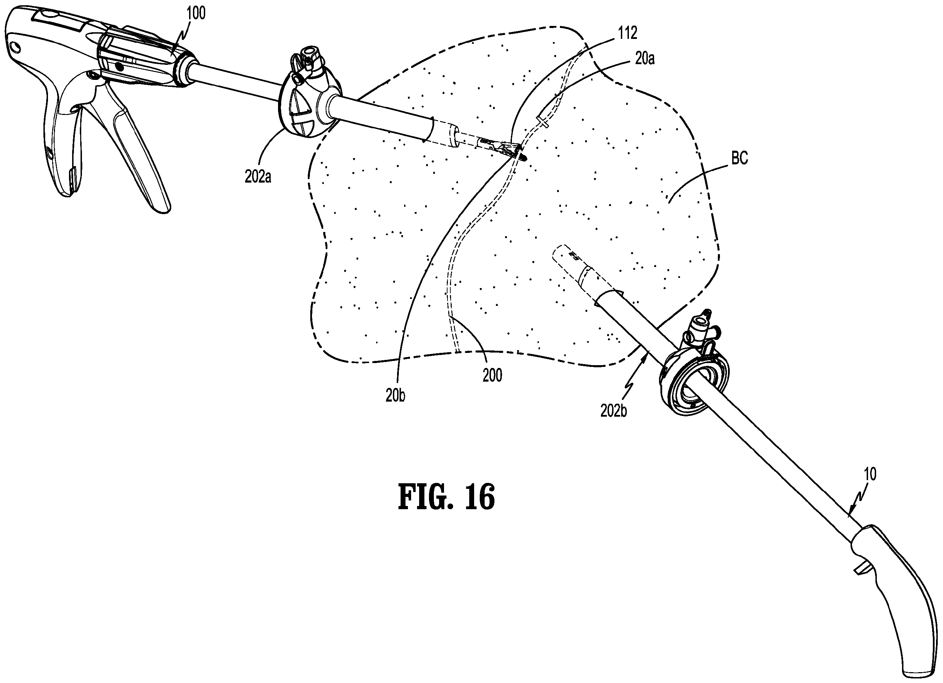

FIGS. 12-16 illustrate use of the loading device 10 and the clip applier 100 during a surgical procedure that requires more than one ligation clip 20 to ligate a body vessel 200. During the surgical procedure, the clip applier 100 and the loading device 10 are inserted through separate incisions "I" into a patient "P" to access a body cavity "BC" (FIG. 14). (Steps 300 and 302 in FIG. 12.) It is envisioned that cannulas 202a and 202b can be positioned within the incisions "I" and the clip applier 100 and the loading device 10 can be inserted through the cannulas 202a and 202b. In aspects of the disclosure, the clip applier 100 is loaded with a first clip 20a (FIG. 14) when the clip applier 100 is inserted through the cannula 202a. After the clip applier 100 is inserted through the cannula 202a, the clip applier 100 is manipulated to position the jaws 112 of the clip applier 100 about the body vessel 200 (FIG. 14) and the clip applier 100 is actuated to close the ligation clip 20a about the body vessel 200. (Step 304 in FIG. 12.) After the ligation clip 20a is closed about the body vessel 200, the jaws 112 of the clip applier 100 are removed from about the closed ligation clip 20a and are inserted into the distal end of the loading device 10 (FIG. 15) in the manner described above to retrieve a second ligation clip 20b (FIG. 16) from the loading device 10. (Step 306 in FIG. 12.) Once the second ligation clip 20b is supported between the jaws 112 of the clip applier 100, the jaws 112 of the clip applier 100 are withdrawn from the loading device 10 and the clip applier 100 is manipulated to reposition the jaws 112 of the clip applier 100 about the body vessel 200 (FIG. 16) (or about a different body vessel). (Step 308 in FIG. 12.) The clip applier 100 can now be actuated to close the second ligation clip 20b about the body vessel 200. This process can be repeated to reload the clip applier 100 after placement of each ligation clip 20. (Step 310 in FIG. 12.) In order to advance the clips 20a-b within the loading device 10 to allow the clips 20 within the loading device 10 to be retrieved by the clip applier 100, the actuator 18 of the loading device 10 must be operated to advance the clips 20a-b within the loading device 10.

It is envisioned that the clip applier 10 can be inserted through the cannula 202a without a ligation clip 20 supported between the jaws 112 of the clip applier 10. In such a case, the jaws 112 of the clip applier 100 can be inserted into the loading device 10 to retrieve a clip 20a-b prior to placement of the first ligation clip 20a about the body vessel 200. Inserting the clip applier 100 through the cannula 202a without a clip 20a-b loaded between the jaws 112 may allow the clip applier 100 to be inserted through a smaller diameter cannula.

Persons skilled in the art will understand that the devices and methods specifically described herein and illustrated in the accompanying drawings are nonlimiting exemplary aspects of the disclosure. It is envisioned that the elements and features illustrated or described in connection with one exemplary embodiment may be combined with the elements and features of another without departing from the scope of the present disclosure. As well, one skilled in the art will appreciate further features and advantages of the disclosure based on the above-described aspects of the disclosure. Accordingly, the disclosure is not to be limited by what has been particularly shown and described, except as indicated by the appended claims.

## Claims

1. An intraoperative clip loading device (10) comprising:
an elongate body (14) having a proximal portion (14b) and a distal portion (14a) and defining a longitudinal axis, the elongate body (14) including an outer tube (30) having a distal portion (30a) defining an opening and including an inner wall(44);
clip retainers (34) supported on the inner wall (44) of the outer tube (30), the clip retainers (34) defining clip retention pockets (56) along the longitudinal axis of the elongate body (14), wherein each of the clip retainers (34) includes a longitudinal portion (52b), wherein each of the longitudinal portions (52b) includes a flexible tab (58) having a stop surface (60);
a plurality of ligation clips (20), each of the plurality of ligation clips (20) being supported within one of the clip retention pockets (56) and including a first beam (22), a second beam (24), and a hinge portion (26) coupling the first beam (22) to the second beam (24), each of the first and second beams (22, 24) including spaced bosses (28), wherein the stop surfaces (60) of the flexible tabs (58) of the clip retainers (34) are positioned to engage the hinge portions (26) of the plurality of ligation clips (20) to prevent proximal movement of the plurality of ligation clips (20) within the outer tube (30);
clip locking arms (36) supported within the distal portion (30a) of the outer tube (30), the clip locking arms (36) formed of a resilient material, the clip locking arms (36) each including a concavity (74) configured to receive the spaced bosses (28) of a respective one of the first and second beams (22, 24) of the ligation clips (20);
a clip advancing member (32) including an elongate member (64) and a plurality of resilient fingers (66), each resilient finger (66) of the plurality of resilient fingers (66) engaging a respective one of the plurality of ligation clips (20), the clip advancing member (32) movable within the outer tube (30) from a retracted position to an advanced position to move the plurality of clips (20) distally within the outer tube (30); and
a guide member (38) positioned on the distal portion (30a) of the outer tube (30), the guide member (38) including diametrically opposed channels (42) configured to receive jaws (112) of a clip applier (100), wherein the clip locking arms (36) are aligned with the opposed channels (42) in the guide member (38), wherein the clip retainers (34) are formed of a resilient material, **characterised in that** each of the clip retainers (34) includes a base portion (50) secured to the inner wall (44) of the outer tube (30), the clip retainers (34) positioned along opposite sides of the outer tube (30), each of the clip retainers (34) aligned with another of the clip retainers (34) such that two diametrically opposed clip retainers (34) cooperate to define one of the clip retention pockets (56),
wherein each of the clip retainers (34) includes a transverse portion (52a) that interconnects the base portion (50) of the clip retainers (34) and the longitudinal portion (52b) of the clip retainers (34).

2. The intraoperative loading device of claim 1, further including a handle assembly (12) including a handle grip (16) and an actuator (18), the actuator (18) being coupled to the clip advancing member (32) such that movement of the actuator (18) moves the clip advancing member (32) between its retracted and advanced positions.

3. The intraoperative loading device of claim 1 or 2, wherein each of the flexible tabs (58) is secured to the longitudinal portion (52b) of a respective one of the clip retainers (34) in cantilevered fashion; and/or wherein the clip locking arms (36) are secured to the inner wall (44) of the outer tube (30) in cantilevered fashion, each of the clip locking arms (36) including a proximal portion (70) and a distal portion (72), the concavities (74) being formed in the distal portion (72) of the clip locking arms (36).

4. The intraoperative loading device of claim 3, wherein the outer tube (30) defines cutouts (76) that are aligned with the distal portions (72) of the clip locking arms (36), the distal portions (72) of the clip locking arms (36) being deflectable outwardly into the cutouts (76) to receive one of the plurality of ligation clips (20).

5. The intraoperative loading device of any of claims 1 to 4, wherein the resilient fingers (66) of the clip advancing member (32) extend inwardly in a distal direction from the elongate member (64) of the clip advancing member (32) into engagement with one of the first and second beam (22, 24) of a respective one of the plurality of ligation clips (20).

## Patentansprüche

1. Intraoperative Klammerladevorrichtung (10), umfassend:
einen länglichen Körper (14), der einen proximalen Abschnitt (14b) und einen distalen Abschnitt (14a) aufweist und eine Längsachse definiert, wobei der längliche Körper (14) ein äußeres Rohr (30) einschließt, der einen distalen Abschnitt (30a) aufweist, der eine Öffnung definiert, und eine Innenwand (44) einschließt;
Klammerhalter (34), die an der Innenwand (44) des äußeren Rohrs (30) gestützt werden, wobei die Klammerhalter (34) entlang der Längsachse des länglichen Körpers (14) Klammerhaltetaschen (56) definieren, wobei jeder der Klammerhalter (34) einen Längsabschnitt (52b) aufweist, wobei jeder der Längsabschnitte (52b) eine flexible Lasche (58) einschließt, die eine Anschlagoberfläche (60) aufweist;
eine Vielzahl von Ligaturklammern (20), wobei jede der Vielzahl von Ligaturklammern (20) innerhalb einer der Klammerhaltetaschen (56) gestützt wird und einen ersten Träger (22), einen zweiten Träger (24) und einen Scharnierabschnitt (26) aufweist, der den ersten Träger (22) mit dem zweiten Träger (24) verbindet, wobei jeder des ersten und des zweiten Trägers (22,24) beabstandete Vorsprünge (28) aufweisen, wobei die Anschlagoberflächen (60) der flexiblen Laschen (58) der Klammerhalter (34) positioniert sind, um mit den Scharnierabschnitten (26) der Vielzahl von Ligaturklammern (20) in Eingriff zu stehen, um eine proximale Bewegung der Vielzahl von Ligaturklammern (20) innerhalb des äußeren Rohrs (30) zu verhindern;
Klammerverriegelungsarme (36), die innerhalb des distalen Abschnitts (30a) des äußeren Rohrs (30) gestützt werden, wobei die Klammerverriegelungsarme (36) aus einem elastischen Material ausgebildet sind, wobei die Klammerverriegelungsarme (36) jeweils eine Konkavität (74) aufweisen, die konfiguriert ist, um die beabstandeten Vorsprünge (28) eines jeweiligen einen des ersten und des zweiten Trägers (22, 24) der Ligaturklammern (20) aufzunehmen;
ein Klammervorschubelement (32), das ein längliches Element (64) und eine Vielzahl von elastischen Fingern (66) umfasst, wobei jeder elastische Finger (66) der Vielzahl von elastischen Fingern (66) mit einer jeweiligen einen der Vielzahl von Ligaturklammern (20) in Eingriff steht, wobei das Klammervorschubelement (32) innerhalb des äußeren Rohrs (30) von einer zurückgezogenen Position in eine vorgeschobene Position bewegbar ist, um die Vielzahl von Klammern (20) distal innerhalb des äußeren Rohrs (30) zu bewegen; und ein Führungselement (38), das an dem distalen Abschnitt (30a) des äußeren Rohrs (30) positioniert ist, wobei das Führungselement (38) diametral gegenüberliegende Kanäle (42) einschließt, die konfiguriert sind, um Backen (112) eines Klammeranbringers (100) aufzunehmen, wobei die Klammerverriegelungsarme (36) mit den gegenüberliegenden Kanälen (42) in dem Führungselement (38) ausgerichtet sind, wobei die Klammerhalter (34) aus einem elastischen Material ausgebildet sind, **dadurch gekennzeichnet, dass** jeder der Klammerhalter (34) einen Basisabschnitt (30) umfasst, der an der Innenwand (44) des äußeren Rohrs (30) gesichert ist, die Klammerhalter (34) entlang gegenüberliegender Seiten des äußeren Rohrs (30) positioniert sind, jeder der Klammerhalter (34) mit einem anderen der Klammerhalter (34) derart ausgerichtet ist, dass zwei diametral gegenüberliegende Klammerhalter (34) zusammenwirken, um eine der Klammerhaltetaschen (56) zu definieren, wobei jeder der Klammerhalter (34) einen Querabschnitt (32a) aufweist, der den Basisabschnitt (50) der Klammerhalter (34) und den Längsabschnitt (32b) der Klammerhalter (34) miteinander verbindet.

2. Intraoperative Ladevorrichtung nach Anspruch 1, die ferner eine Griffanordnung (12) einschließt, die einen Haltegriff (16) und ein Betätigungselement (18) einschließt, wobei das Betätigungselement (18) mit dem Klammervorschubelement (32) derart gekoppelt ist, dass eine Bewegung des Betätigungselements (18) das Klammervorschubelement (32) zwischen seiner zurückgezogenen und vorgeschobenen Position bewegt.

3. Intraoperative Ladevorrichtung nach Anspruch 1 oder 2, wobei jede der flexiblen Laschen (58) an dem Längsabschnitt (52b) eines jeweiligen einen der Klammerhalter (34) in freitragender Weise gesichert ist; und/oder wobei die Klammerverriegelungsarme (36) an der Innenwand (44) des äußeren Rohrs (30) in freitragender Weise gesichert sind, wobei jeder der Klammerverriegelungsarme (36) einen proximalen Abschnitt (70) und einen distalen Abschnitt (72) einschließt, wobei die Konkavitäten (74) in dem distalen Abschnitt (72) der Klammerverriegelungsarme (36) ausgebildet sind.

4. Intraoperative Ladevorrichtung nach Anspruch 3, wobei das äußere Rohr (30) Ausschnitte (76) definiert, die mit den distalen Abschnitten (72) der Klammerverriegelungsarme (36) ausgerichtet sind, wobei die distalen Abschnitte (72) der Klammerverriegelungsarme (36) nach außen in die Ausschnitte (76) biegbar sind, um eine der Vielzahl von Ligaturklammern (20) aufzunehmen.

5. Intraoperative Ladevorrichtung nach einem der Ansprüche 1 bis 4, wobei sich die elastischen Finger (66) des Klammervorschubelements (32) in einer distalen Richtung von dem länglichen Element (64) des Klammervorschubelements (32) nach innen in Eingriff mit einem des ersten und des zweiten Trägers (22, 24) einer jeweiligen einen der Vielzahl von Ligaturklammern (20) erstrecken.

## Revendications

1. Dispositif de chargement de clip peropératoire (10) comprenant :
un corps allongé (14) ayant une partie proximale (14b) et une partie distale (14a) et définissant un axe longitudinal, le corps allongé (14) comportant un tube externe (30) ayant une partie distale (30a) définissant une ouverture et comportant une paroi interne (44) ;
des éléments de retenue de clip (34) supportés sur la paroi interne (44) du tube externe (30), les éléments de retenue de clip (34) définissant des poches de rétention de clip (56) le long de l'axe longitudinal du corps allongé (14), dans lequel chacun des éléments de retenue de clip (34) comporte une partie longitudinale (52b), dans lequel chacune des parties longitudinales (52b) comporte une languette flexible (58) ayant une surface de butée (60) ;
une pluralité de clips de ligature (20), chacun de la pluralité de clips de ligature (20) étant supporté à l'intérieur d'une des poches de rétention de clip (56) et comportant une première poutre (22), une seconde poutre (24) et une partie charnière (26) accouplant la première poutre (22) à la seconde poutre (24), chacune parmi la première et la seconde poutre (22, 24) comportant des bossages espacés (28), dans lequel les surfaces de butée (60) des languettes flexibles (58) des éléments de retenue de clip (34) sont positionnées pour venir en prise avec les parties charnières (26) de la pluralité de clips de ligature (20) pour empêcher un mouvement proximal de la pluralité de clips de ligature (20) à l'intérieur du tube externe (30) ;
des bras de verrouillage de clip (36) supportés à l'intérieur de la partie distale (30a) du tube externe (30), les bras de verrouillage de clip (36) formés d'un matériau élastique, les bras de verrouillage de clip (36) comportant chacun une concavité (74) conçue pour recevoir les bossages espacés (28) de l'une respective parmi la première et la seconde poutre (22, 24) de clips de ligature (20) ;
un élément d'avancement de clip (32) comportant un élément allongé (64) et une pluralité de doigts élastiques (66), chaque doigt élastique (66) de la pluralité de doigts élastiques (66) venant en prise avec un clip respectif de la pluralité de clips de ligature (20), l'élément d'avancement de clip (32) pouvant être déplacé à l'intérieur du tube externe (30) d'une position rétractée à une position avancée afin de déplacer la pluralité de clips (20) de manière distale à l'intérieur du tube externe (30) ; et
un élément de guidage (38) positionné sur la partie distale (30a) du tube externe (30), l'élément de guidage (38) comportant des canaux diamétralement opposés (42) conçus pour recevoir des mâchoires (112) d'un applicateur de clip (100), dans lequel les bras de verrouillage de clip (36) sont alignés avec les canaux opposés (42) dans l'élément de guidage (38), dans lequel les éléments de retenue de clip (34) sont formés d'un matériau élastique, **caractérisé en ce que** chacun des éléments de retenue de clip (34) comporte une partie de base (50) fixée à la paroi interne (44) du tube externe (30), les éléments de retenue de clip (34) positionnés le long de côtés opposés du tube interne (30), chacun des éléments de retenue de clip (34) alignés avec un autre des éléments de retenue de clip (34) de telle sorte que deux éléments de retenue de clip (34) diamétralement opposés coopèrent pour définir une des poches de rétention de clip (56),
dans lequel chacun des éléments de retenue de clip (34) comporte une partie transversale (52a) qui relie la partie de base (50) des éléments de retenue de clip (34) et la partie longitudinale (52b) des éléments de retenue de clip (34).

2. Dispositif de chargement peropératoire selon la revendication 1, comportant en outre un ensemble poignée (12) comportant une poignée (16) et un actionneur (18), l'actionneur (18) étant accouplé à l'élément d'avancement de clip (32) de telle sorte que le mouvement de l'actionneur (18) déplace l'élément d'avancement de clip (32) entre ses positions rétractée et avancée.

3. Dispositif de chargement peropératoire selon la revendication 1 ou 2, dans lequel chacune des languettes flexibles (58) est fixée à la partie longitudinale (52b) d'un élément de retenue respectif des éléments de retenue de clip (34) en porte-à-faux ; et/ou dans lequel les bras de verrouillage de clip (36) sont fixés à la paroi interne (44) du tube externe (30) en porte-à-faux, chacun des bras de verrouillage de clip (36) comportant une partie proximale (70) et une partie distale (72), les concavités (74) étant formées dans la partie distale (72) des bras de verrouillage de clip (36).

4. Dispositif de chargement peropératoire selon la revendication 3, dans lequel le tube externe (30) définit des découpes (76) qui sont alignées avec les parties distales (72) des bras de verrouillage de clip (36), les parties distales (72) des bras de verrouillage de clip (36) pouvant être déviées vers l'extérieur dans les découpes (76) pour recevoir l'un de la pluralité de clips de ligature (20).

5. Dispositif de chargement peropératoire selon l'une quelconque des revendications 1 à 4, dans lequel les doigts élastiques (66) de l'élément d'avancement de clip (32) s'étendent vers l'intérieur dans une direction distale à partir de l'élément allongé (64) de l'élément d'avancement de clip (32) pour venir en prise avec l'un parmi la première et la seconde poutre (22, 24) d'un clip de ligature respectif parmi la pluralité de clips de ligature (20).
